# EUROPEAN PATENT APPLICATION

(11) **EP 4 253 523 A1**
(43) Date of publication of application: **04.10.2023**
(21) Application number: 22166252.1
(22) Date of filing: 01.04.2022
(51) Int. Cl.: C12M 3/00, C12M 3/06, C12M 1/00, C12M 1/24

(54) **AUTOMATED INCUBATOR SHAKER COMPARTMENT**

(71) Applicant: Selexis S.A., 1228 Plan-les-Ouates (CH)
(72) Inventor: Regamey, Pierre-Olivier, 1228 Plan-les-Ouates (CH); Voisin-Martiné, Alexandra, 1228 Plan-les-Ouates (CH); Girod, Pierre-Alain, 1228 Plan-les-Ouates (CH); Aussems, Camille, 1228 Plan-les-Ouates (CH)
(74) Representative: Wenger, Joel-Théophile

(57) **Abstract**

The present invention is directed to a set (7) for maintaining and transporting at least one cell culture flask arranged in at least one cell culture flask holder, and to the use of this set. The present invention is directed to an incubator shaker comprising said set (7).

## Description

### Field

The present invention relates to the field of cell culture platforms and in particular to the automated cell culture platforms comprising a shaker incubator and means for handling labware in automated cell culture platforms. In particular, the present invention relates to an automated incubator shaker compartment for use in an automated culture platform.

### Background

Cell culture refers to a laboratory method that enable the growth of eukaryotic or prokaryotic cells in physiological conditions. It is a method used to study for example tissue growth and maturation, virus biology and vaccine development, the role of genes in disease and health, and the use of large-scale hybrid cell lines to generate biopharmaceuticals. The experimental applications of cultured cells are as diverse as the cell types that can be grown *in vitro.* One specific example of a cell culture method is cell passaging (or subculturing), that involves the removal of a culture medium and transfer of cells from a previous culture into fresh growth medium. This procedure enables the further propagation of the cell line or cell strain.

The methods of cell culturing require use of dedicated laboratory equipment and precise multistep cells' manipulation. Manual procedures are difficult to adopt to GMP (good manufacturing practice) and GLP (good laboratory practice), and they should thus generally not be used in biotech or pharmaceutical enterprises. Therefore, some processes/steps of cell culturing are automated. One example of automated cell passaging platform exists for microtiter plates (MTP) culture formats as described by the University of Luxembourg *(Automated Cell Culture Maintenance and High Throughput*/*High Content Screening Platform; https:*//*wwwen.uni.lu*/*lcsb*/*research*/*translational_neuroscience*/*automation_platform.).* This is an example of a high throughput/high content platform for maintaining induced pluripotent stem cells in culture, differentiating these cells into neurons while performing chemical or viral-vector-based genetic screenings.

One example of a device used for cell culture is a shaking (or a shaker) incubator that is a laboratory equipment that allows for incubation and shaking of the cell cultures. A typical incubator shaker comprises at least a chamber fitted with door, a shaking tray fitted with a motor and means for shaking control, as well as means for temperature control. The loading and unloading of shaking trays are typically done manually and include opening of the door. The process may be automated such that the incubators' door opening, and closing is automated and a robot arm, placed outside of the incubator, can perform a loading and unloading of shaking trays. The automated process is typically compatible with one type of labware, typically microtiter plates (MTP). In this example, loading and unloading of shaking trays must be done when the door is open, and the robot arm mimics a manual action of manipulating e.g., MTP. Cell cultivation within the incubator requires maintenance of the temperature, humidity and composition of the defined gas atmosphere within the incubator. If the incubator is open during cultivation, the temperature, humidity and composition of the gas atmosphere within the incubator and consequently within cultures can change and negatively impact cells. Further, typically, a shaking incubator may be provided with a universal tray that can be fitted with fixed on the tray clamps for labware such as clamps for Erlenmeyer flasks, test tube holders, microtiter plates, or other custom-made holders. In another example, such a tray may be fitted with pins, so that flasks are held in position by these pins. In another example, such a tray may be fitted with sticky strips, wherein a labware is placed directly on the sticky strip without the need for any other support. Most of this solution are not compatible with loading and unloading of shaking trays performed with an automated robot arm.

It is an object of the invention to provide improved devices and methods for automated incubation and handling of the cell cultures contained in a suitable labware for cell cultures.

### Summary

The present invention is based on the selection of the favourable labware for maintaining cell cultures, preferably for use in cell passaging method for seedtrain cultivation. In particular, Erlenmeyer flasks or bioreactor tubes are suitable labware for maintaining cell cultures.

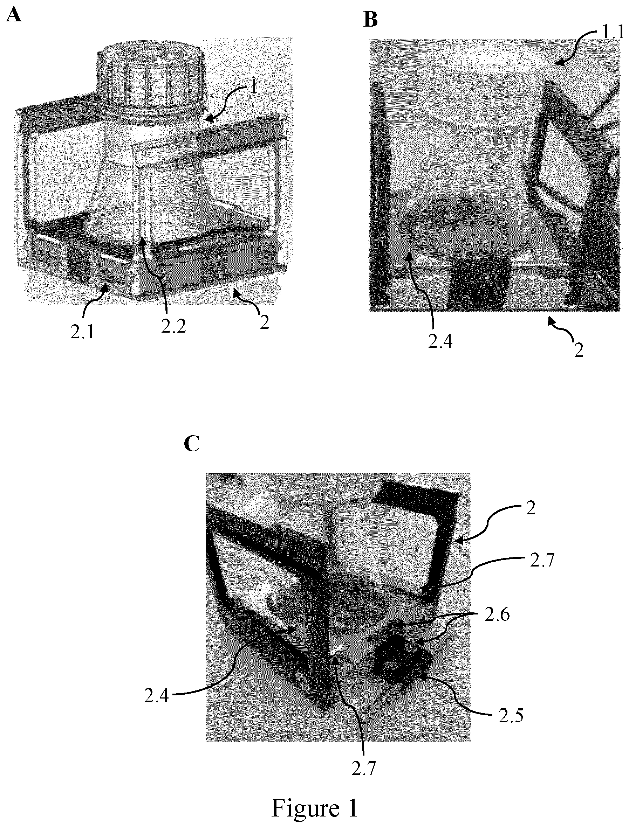

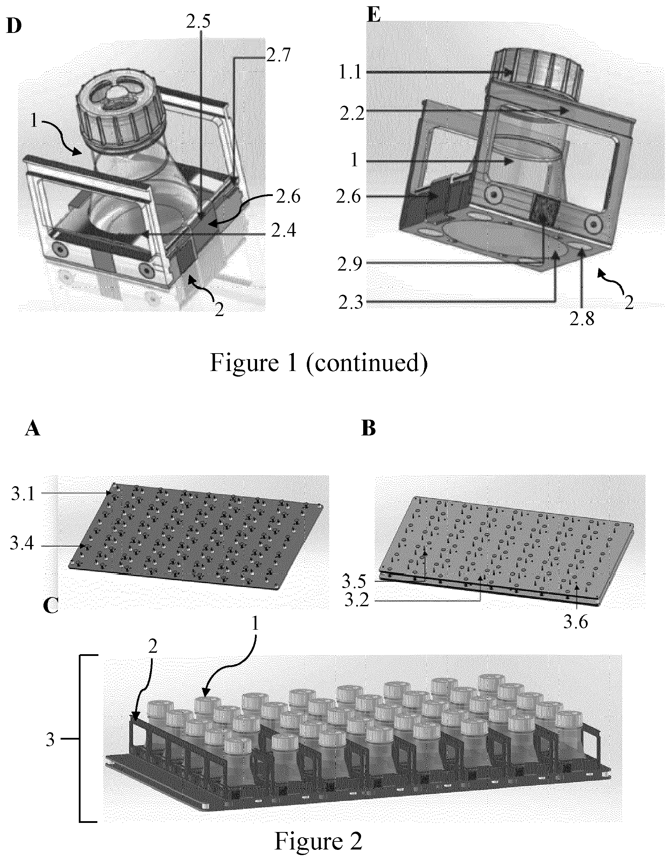

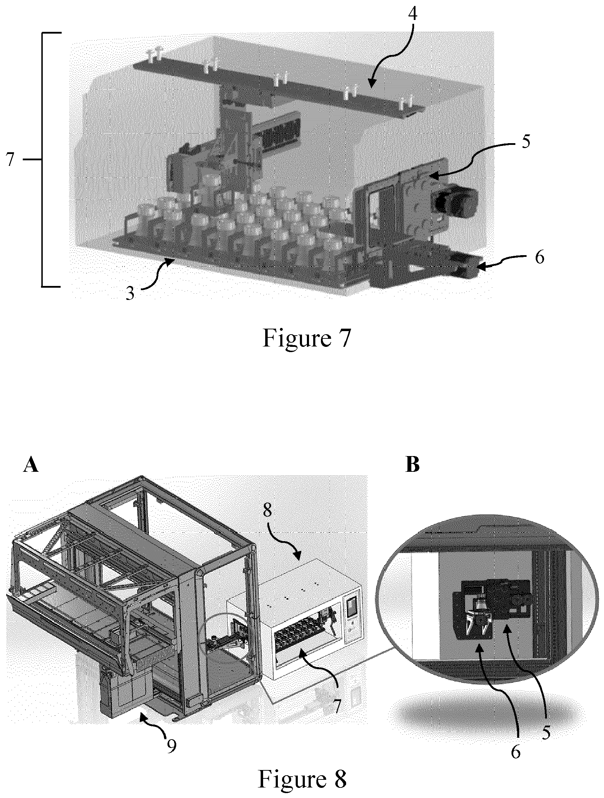

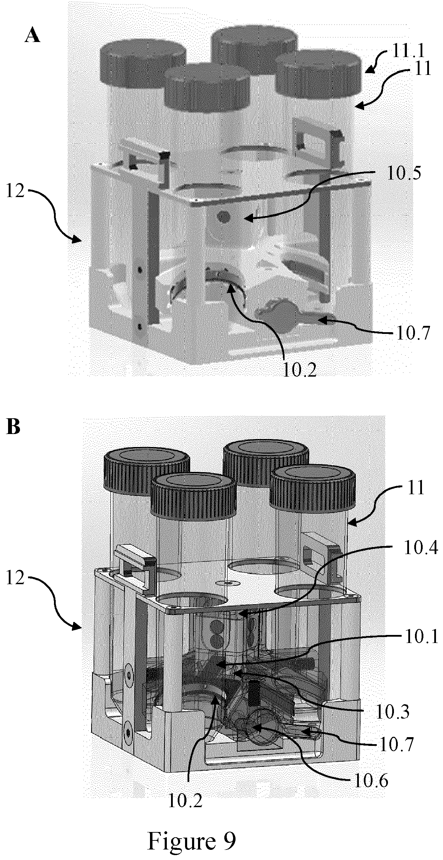

The present invention is based on providing a holder (or a basket) (2, 12) for maintaining specific labware that contains cell cultures (e.g., a flask (1) as in fig. 1; or a bioreactor tube (11) as in fig. 9). The present invention is based on providing an automated set (7) for maintaining and transporting cell culture labware (e.g., flasks, bioreactor tubes), that is suitable for an incubator shaker compartment, and comprising an incubator shaker tray (3), a 3-axis motorized stage (4), a sliding door (5), and a transfer shuttle (6) (fig. 7). The present invention relates to a shaking incubator having an interior space in which a plurality of baskets for cell culture labware (e.g., flasks, bioreactor tubes) may be arranged on a shaking tray. The shaking incubator has a chamber designed to be arranged with the set for maintaining and transporting cell culture labware (e.g., flasks, bioreactor tubes) holders. The present invention is particularly adopted for use in an automated cell passaging platform.

The present invention allows manipulating cell culture containing labware individually and independently of each other in a shaker incubator via a cell culture labware holder which is connected detachably to a shaking tray.

In the case of parallel cultivation of a plurality of cultures in one shaker incubator, one of the flasks holders is taken from the incubator via the set of the invention and this movement has limited influence on the environment of the cultures in the other labware. Although the temperature changes slightly on opening of the automated sliding door of the set, this small temperature change does have minimal, or no influence on the cultures in the other labwares. The set of the invention facilitates the loading and unloading of shaking trays and therefore enhances the ergonomic handling of cultures.

In one embodiment is provided a set for maintaining and transporting at least one cell culture flask arranged in at least one cell culture flask holder, wherein the set is designed to be arranged in an incubator shaker chamber, and the set comprising
- an incubator shaker tray designed to be mounted on the inside of the incubator shaker chamber, that comprises at least one guiding element arranged for maintaining each cell culture flask holder,
- a 3-axis motorized stage designed to be mounted on the inner side of a top wall of the incubator shaker chamber, that comprises X-axis, Y-axis and Z-axis and three linear motors for driving the three axis and means for gripping the cell culture flask holder,
- a sliding door designed to be mounted within a side wall of the incubator shaker chamber, that comprises a door panel and at least one motor for driving the door movement,
- a transfer shuttle designed to be mounted on the outer side of a side wall of the incubator shaker chamber, that comprises a sliding frame, rails, at least two belts arranged on the sliding frame, a cell culture flask holder plate and a motor for driving sliding movement of the plate on the rails.

In another embodiment is provided the set for maintaining and transporting at least one cell culture flask according to the invention, wherein the cell culture flask is a 125ml Erlenmeyer flask or a bioreactor tube.

In another embodiment is provided the set according to the invention, wherein the means for gripping the cell culture flask holder are attached to Z-axis of the 3-axis motorized stage and comprise one parallel gripper with an adjustable prehension force, equipped with jaws and fingers for detachable connection to the flask holder.

In another embodiment is provided the set according to the invention, wherein the motor for driving the door movement of the sliding door comprises a stepper motor for driving a sliding movement and a stepper motor for driving a press movement of the door panel.

In another embodiment is provided the set according to the invention, wherein the sliding door further comprises a precision sensor for detecting a sliding movement of the door panel and a precision sensor for detecting press movement of the door panel.

In another embodiment is provided the set according to the invention, wherein the sliding door further comprises a temperature probe, a heating pad, a heating ribbon and a controller, and wherein the temperature probe is controlling the heating pad and the heating ribbon through the controller.

In another embodiment is provided the set according to the invention, wherein the sliding door further comprises a silicone o-ring fitted between the door frame and the door panel.

In another embodiment is provided the set according to the invention, wherein the transfer shuttle further comprises an amplified precision sensor for detecting a transfer shuttle home position, a sensor for detecting of loading position of the cell culture flask holder, and a sensor for detecting of unloading position of the cell culture flask holder.

In another embodiment is provided the set according to the invention, that further comprises a cell culture flask holder, and the cell culture flask holder comprises a base, two handles arranged perpendicular to the base and opposite of each other, a locking mean for maintaining the cell culture flask and a heat conductive pad arranged on the base of the holder.

In another embodiment is provided the set according to the invention, wherein the cell culture flask holder is further comprising at least two magnets arranged on the outer side of the cell culture flask holder base that provide attachment means to the incubator shaker comprising permanent electro-magnets.

In another embodiment is provided the set according to the invention, wherein the cell culture flask holder's locking mean comprises a clamping mean, and a safety latch arranged on the cell culture flask holder base.

In another embodiment is provided the set according to the invention, wherein the cell culture flask holder's locking mean comprises four guillotines each holding a toothed jaw and arranged on the cell culture flask holder base.

In another embodiment is provided a use of the set according to the invention for maintaining and transporting at least one cell culture flask arranged in at least one cell culture flask holder compatible with the set.

In another embodiment is provided an incubator shaker comprising the set according to the invention.

In another embodiment is provided an automated cell passaging platform comprising a liquid handling system, at least one robotized arm, at least one automated hotel, an automated refrigerated incubator, an automated microplate centrifuge with automated loader, a cell counting device and at least one shaker incubator, and optionally an automated orbital shaker incubator, characterised in that the at least one shaker incubator is designed to be arranged with the set for maintaining and transporting at least one cell culture flasks according to the invention.

### Description of the figures

**Figure 1** shows an example of a flask basket for 125ml Erlenmeyer flask; panels A, D, E show drawings; panels B, C show pictures. The flask basket (2) for 125ml Erlenmeyer flask (1) comprises a base (2.1), two handles (one handle - 2.2), a heat conductive pad (2.3), a toothed spring with a horseshoe shape (2.4), a safety latch (2.5), latching magnets (2.6), a latching embossment (2.7), magnetic pads (2.8) and a RFID tag or QR-code (2.9).
**Figure 2** shows an example of an incubator shaker tray (3). Panel A shows a lower plate (3.1) with eighty permanent electro-magnets (3.4). Panel B shows assembled lower plate (3.1) with upper plate (3.2) (a tray locker 3.3 is not shown). The upper plate (3.2) has eighty centering pins (3.5) and forty RFID antenna holes (3.6). Panel C shows an incubator shaker tray (3) with forty flask holders (2) for 125ml Erlenmeyer flask (1) arranged in 8x5 array.

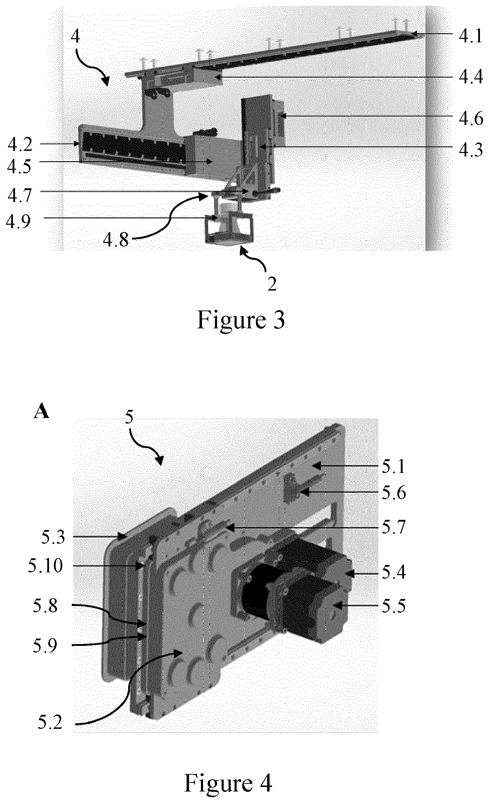

**Figure 3** shows an example of a 3-axis motorized stage (4) that comprises X-axis (4.1), Y-axis (4.2), Z-axis (4.3), a linear motor (4.4) for driving the X-axis (4.1) (not visible), a linear motor (4.5) for driving the Y-axis (4.2) (not visible), a linear motor (4.6) for driving the Z-axis (4.3) (not visible), a parallel gripper (4.7) with jaws (4.8) and fingers (4.9).

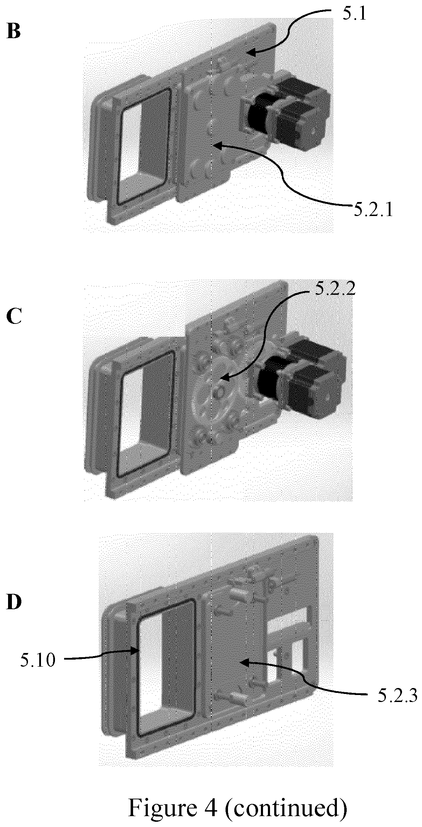

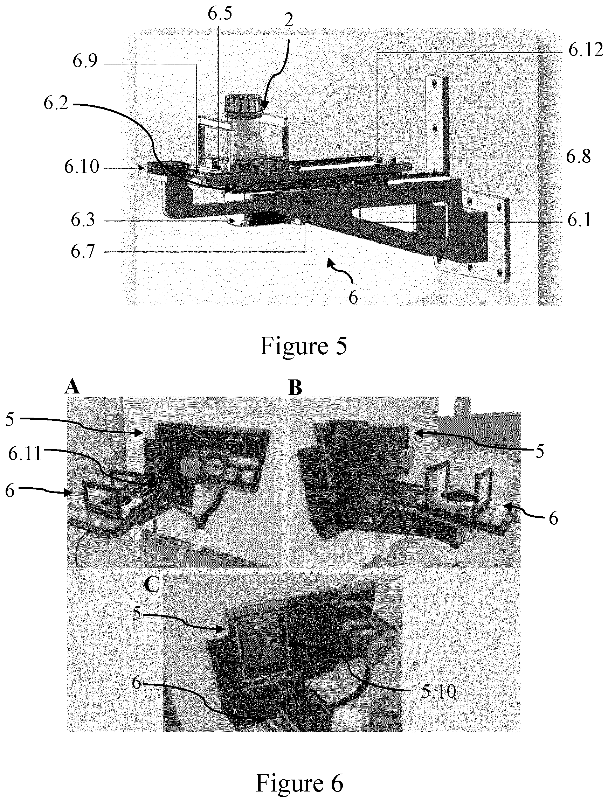

**Figure 4** shows an example of a sliding door (5). Panel A: shows an example of a sliding door (5) that comprise a door frame (5.1) and a door panel (5.2), a door tunnel (5.3), sliding movement driving stepper motor (5.4) and press movement driving stepper motor (5.5), a precision sensor for sliding movement (5.6) and a precision sensor for press movement (5.7), a temperature probe (5.8) (not visible), a heating pad (5.9) (not visible), an o-ring (5.10) (not visible; marked in fig. 4D or 6C) and a heating ribbon (5.11) (not visible). Panels B-D shows three parts of the door panel (5.2) that are front panel (5.2.1) (fig. 4B shows external view of opened sliding door), main body (5.2.2) (fig. 4C shows central parts of opened sliding door) and pressing panel (5.2.3) (fig. 4D).
**Figure 5** shows an example of a transfer shuttle (6) that comprises a sliding frame (6.1) with two upper and two lower rails (6.2), sliding movement driving stepper motor (6.3), a pinion (6.4) (not visible), a flask holder plate (6.5), a flask holder plate (6.5) with two centering pins (6.6) (not visible), a precision sensor for detecting the transfer shuttle home position (6.7), a sensor for detecting of the loading position of the flask holder (6.8) and a sensor for detecting of the unloading position of the flask holder (6.9), a presence laser detector (6.10), belts (6.11) (not visible; marked in fig. 6A), a double extension plate (6.12) and a ball screw (6.13) (not visible).
**Figure 6** shows an example of a transfer shuttle (6) designed to be mounted on the outer side of a side wall of an incubator shaker chamber below the sliding door (5). Panel A shows the belts (6.11) of the transfer shuttle (6) and Panel C shows the o-ring (5.10) of the sliding door (5).
**Figure 7** shows an example of a set (7) for maintaining and transporting cell culture flasks designed to be mounted in the incubator shaker chamber (chamber is marked as shade contour) comprising an incubator shaker tray (3), a 3-axis motorized stage (4), a sliding door (5), and a transfer shuttle (6).
**Figure 8** shows an example of the arrangement between an incubator shaker (8) comprising a set of the invention (7) within an automated cell passaging platform, wherein the automated shaker incubator is integrated with a liquid handling system (9) through a hole/window drilled on walls of the rear cabinet (not marked) and connected via the transfer shuttle (6) (Panel A). Panel B shows the sliding door (5) and the transfer shuttle (6) as seen on the outer side of a side wall of an incubator shaker for transporting the flask holder to a liquid handling system (9).
**Figure 9** shows an example of a flask basket (12) for 50ml bioreactor tube (11) with the locking means for four bioreactor tubes (10). Panels A and B show drawings. The flask basket (12) for 50ml bioreactor tube comprises four guillotines (10.1), eight toothed jaws (10.2), four rail-and-pivots (10.3) (pivot not visible), a plunger (10.4), a plunger holder (10.5), an eccentric jig (10.6), a controlling latch (10.7) and a RFID tag or QR-code (2.9) (not marked).

### List of reference numerals:

1. a flask (e.g., 125ml Erlenmeyer flask); 1.1. a cap (e.g., 125ml Erlenmeyer flask cap); 2. a flask basket; 2.1. a base of a flask basket; 2.2. a handle of a flask basket; 2.3. a heat conductive pad for a flask basket; 2.4-2.7. locking means for Erlenmeyer flask; 2.4. a toothed spring with a horseshoe shape of a flask basket; 2.5. a safety latch; 2.6. latching magnets; 2.7. a latching embossment; 2.8. a magnetic pad; 2.9. a RFID tag or QR-code; 3. an incubator tray; 3.1. a lower plate; 3.2. an upper plate; 3.3. a tray locker; 3.4. permanent electro-magnets; 3.5. centering pins; 3.6. RFID antenna hole; 4. 3-axis motorized stage; 4.1. X-axis; 4.2 Y-axis; 4.3 Z-axis; 4.4. a linear motor for the X-axis (4.1); 4.5. a linear motor for the Y-axis (4.2); 4.6. a linear motor for the Z-axis (4.3); 4.7. a parallel gripper; 4.8. a parallel gripper's jaws; 4.9. a parallel gripper's fingers; 5. a sliding door; 5.1. a door frame; 5.2 a door panel; 5.2.1. front panel of a door panel; 5.2.2. main body of a door panel; 5.2.3. pressing panel of a door panel; 5.3 a door tunnel; 5.4 a sliding movement driving stepper motor; 5.5. a press movement driving stepper motor; 5.6. a precision sensor for sliding movement; 5.7. a precision sensor for press movement; 5.8. a temperature probe; 5.9. a heating pad; 5.10. an o-ring; 5.11. a heating ribbon; 6. a transfer shuttle; 6.1. a sliding frame; 6.2. upper and lower rails; 6.3. a sliding movement driving stepper motor; 6.4. a pinion; 6.5. a flask holder plate; 6.6. a flask holder plate's two centering pins; 6.7. an amplified precision sensor for detecting the transfer shuttle home position; 6.8. a sensor for detecting of the loading position of the flask holder; 6.9. a sensor for detecting of the unloading position of the flask holder; 6.10. a presence laser detector; 6.11. belts; 6.12. a double extension plate; 6.13. a ball screw; 7. a set for maintaining and transporting at least one cell culture flask; 8. an incubator shaker comprising a set of the invention; 9. a liquid handling system; 10.1-10.7. locking means for four bioreactor tubes; 10.1. a guillotine; 10.2. a toothed jaw; 10.3. a rail-and-pivot; 10.4. a plunger; 10.5. a plunger holder; 10.6. an eccentric jig; 10.7. a controlling latch. 11. a bioreactor tube; 11.1. a bioreactor tube cap (e.g., filter cap); 12. a flask basket for bioreactor tubes

### Detailed description

The term "a shaking incubator" or "a shaker incubator" or "incubator shaker" (terms used interchangeably) refers to a laboratory equipment that is combining incubation and shaking functions. A typical incubator shaker comprises at least a chamber (preferably made from stainless steel or painted stainless steel), a door comprising a door glass and frame (herein a main door), a tray, a motor that moves the tray in a shaking motion, means for shaking control (e.g., speed, frequency), means for heating/cooling and means for temperature (heating/cooling) control. In addition, the incubator shaker may comprise means for regulating humidity and means for humidity control, means for regulating CO₂ level and means for CO₂ control and other environmental parameters control (e.g., light). The incubator shaker can be used for variety of applications in the fields of biology, chemistry, biochemistry, pharmacology, and the like, including but not limited to any type of cell culture cultivation (including bacterial cultures, tissue cultures, and yeast), protein expression, solubility studies, or general mixing. Depending on the laboratory set-up and designated use, the shaker incubators may range from compact, benchtop systems to large-capacity, floor-stackable and the like, with various means for temperature control and means for shaking speed control. The term "a flask" or "a shaker flask" refers to any type of labware suitable for containing liquids or cell cultures and suitable for mixing, heating, cooling, incubation, filtration, storage, and other liquid-handling processes. A shaker flask may be an Erlenmeyer flask, also known as a conical flask or a titration flask, which features a flat bottom, a conical body, and a cylindrical neck. Another example of a shaker flask may be a Fernbach flask, which are baffled on the bottom in order to maximize oxygen transfer to the culture medium when shaken. Another example of a shaker flask may be a bioreactor tube which can be conical on the bottom and allow smaller working volume of 1-15 mL.

The term "a cell culture" refers to the process by which cells are grown under controlled conditions outside their natural environment. Cells cultured in the lab can be classified into three different types: primary cells, transformed cells, and self-renewing cells. Among the common manipulations carried out on culture cells are media changes, passaging cells, and transfecting cells. "Passaging" (also known as subculture or splitting cells) involves transferring a small number of cells into a new vessel. Cells can be cultured for a longer time if they are split regularly, as it avoids the senescence associated with prolonged high cell density.

The cell passaging may be performed with a use of an automated cell passaging platform. An exemplary automated cell passaging platform may be comprising
- a liquid handling system (such as Hamilton VANTAGE 2.0 liquid handling system),
- at least one robotized arm (such as Precise, PF400 extended),
- at least one automated hotel (such as Liconic LPX740),
- an automated refrigerated incubator (such as Liconic STX44-HCBT),
- optionally, an automated orbital shaker incubator (such as Liconic STX88-ICMISA),
- an automated microplate centrifuge with automated loader (such as Agilent),
- a cell counting device (such as Raman Rxn2-785 Analyzer, Kaiser/Endress&Hauser), and
- at least one (up to four) shaker incubator (such as Kühner, ISF1-ZC Peltier).

### A flask holder

The present invention is based on the selection of the favourable labware for maintaining cell cultures, preferably for use in cell passaging method for seed train cultivation. In particular, Erlenmeyer flasks are suitable labware for maintaining cell cultures. Preferably, 125mL Erlenmeyer flask format provides a sufficient volume of cell culture for the required cell culture applications (20 mL). However, Erlenmeyer flasks are considered as high labware, such as higher than microplates. The size of Erlenmeyer flasks is not compatible with known automated lab equipment. However, Erlenmeyer flasks limits the risk of cross-contamination compared to deep-well plate formats. Preferably, Erlenmeyer flasks are sterile, baffled and threaded flasks with a PTFE (polytetrafluoroethylene) 0.2µm vent cap (i.e., a filtering cap) for increased aeration and can provide an optimal growth of the selected cell lines. Another suitable labware for maintaining cell cultures are bioreactor tubes which are sterile and threaded tubes with a PTFE 0.2µm vent cap (i.e., a filtering cap).

In one embodiment, is provided a holder for a flask, such as 125ml Erlenmeyer flask (1). The flask holder is referred herein as a basket. The terms "a (shaker) flask holder" and "a (shaker) flask basket" may be used interchangeably. In particular, the cell culture labware may be a flask, and the cell culture labware holder (basket) is a flask holder (basket). Since the flask holder is arranged within a shaker incubator it may be referred herein as a shaker flask holder. In one embodiment, the flask maintains CHO-M (Chinese hamster ovary) seed train cell lines (Selexis).

In one embodiment, is provided a flask holder which lateral dimension may be 87.26 ± 0.07 mm width and 85.64 ± 0.08 mm length. The use of the flask holder of this dimension is compatible with known other automated laboratory equipment compatible with MTP labware, such as MTP deck of the VANTAGE 2.0 liquid handling system (Hamilton). Thus, the flask holder as described herein is matched with Society for Biomolecular Screening (SBS) defined dimensions for standard plates.

In one embodiment, is provided a flask holder (2) that comprises at least a base (2.1), two handles (2.2), a heat conductive pad (2.3), a locking means (such as a combination of 2.4, 2.5, 2.6, 2.7). The flask holder (2) may further comprise a magnetic pad (2.8) and identification means (2.9).

Possible elements of the flask holder may be described as follows:
- a base (2.1)

In one embodiment, a flask holder has a base that supports further elements of the holder such as two handles (2.2), a heat conductive pad (2.3), a toothed spring with a horseshoe shape (2.4), a safety latch (2.5), latching magnets (2.6), a latching embossment (2.7), a magnetic pad (2.8) and a RFID tag or QR-code (2.9). In one embodiment, the base can be seen as in Fig. 1A-E. In one embodiment, the base is preferably made of *PEEK* polymer (polyether ether ketone polymer). In general, use of *PEEK* polymer prevents from corrosion inside the incubator shaker compartment.
- a handle (2.2)

In one embodiment one or two handles are arranged on the flask holder base. Preferably, two handles are arranged on the flask holder base, so that the handles are perpendicular to the base and the handles are opposite of each other.

In one embodiment, the handles are rectangle shaped, preferably are hollow, wherein at least one side is shaped to provide a gripping side compatible with a gripping means, such as the gripping means of a 3-axis motorized stage or a 4-axis collaborative robot. In another embodiment, the handles are rectangle shaped, preferably are hollow, wherein at least one side is embossed to provide a gripping side compatible with gripping means, such as the gripping means of a 3-axis motorized stage or a 4-axis collaborative robot. The shape of the handles enables gripping by gripping means (such as the gripping means of a 3-axis motorized stage (4.7) or a gripping means of a 4-axis collaborative robot) and may be referred herein as a gripping handle.

The handles may be attached to the base by any attachment means, such as screws.

In one embodiment, a handle is preferably made of hard anodized aluminium alloy. In general, use of hard anodized aluminium alloy prevents from corrosion inside the incubator shaker compartment.

In one embodiment, the two handles can be arranged as seen in Fig. 1A-E.

In one embodiment, the handles hight may be modified so that to accommodate the labware of different hight and still allow for gripping.
- a heat conductive pad (2.3)

In one embodiment, a heat conductive pad is fitted within a hollow-cylindrical opening on the base of the flask holder and is arranged under a base of the flask.

In one embodiment, a heat conductive pad (2.3) can be seen as in Fig. IE.

The heat conductive pad is arranged so that to provide a direct heating to the flask placed in the flask holder.

The heat conductive pad is an example of means to provide heating to the flask placed in the flask holder.

The heat conductive pad is made of a conductive material that conducts heat, preferably of a material with high thermal conductivity such as metals. Heat transfer occurs when this material is placed on a heated surface, so that this material favours thermal exchanges to e.g., a flask. In one embodiment, a heat conductive pad is preferably made of hard anodized aluminium alloy.
- locking means (for a flask - combination of 2.4, 2.5, 2.6, 2.7 or for a bioreactor tube combination of 10.1, 10.2, 10.3, 10.4, 10.5, 10.6, 10.7)

In one embodiment, locking means are arranged on the flask holder base.

In one alternative embodiment, locking means are suitable for an Erlenmeyer flask, and may comprise at least one of toothed clamping means (such as 2.4) with a latching embossment (such as 2.7) and a latch (such as a safety latch (2.5)) with latching magnets (such as 2.6). The function of this locking means is to maintain the flask blocked from rotation and vertical displacement. This locking means for an Erlenmeyer flask are arranged on the flask holder base as seen in fig. 1

In another alternative embodiment, locking means are suitable for a bioreactor tube flask and comprise four guillotines (10.1) each holding a toothed jaw (10.2) and arranged on the cell culture flask holder base.

In another embodiment, locking means are suitable for a bioreactor tube flask may be a combination of four guillotines (10.1), eight toothed jaws (10.2), four rail-and-pivots (10.3) (pivot not visible), a plunger (10.4), a plunger holder (10.5), an eccentric jig (10.6), a controlling latch (10.7).

The function of this locking means is to maintain up to four bioreactor tubes blocked from rotation and vertical displacement. This locking means for a bioreactor tube are arranged on the flask holder base as seen in fig. 9.
∘ locking means for an Erlenmeyer flask

In one embodiment the locking means for an Erlenmeyer flask are arranged on the holder's base and perpendicular to the base's bottom. The locking means are arranged on the holder's base part that is not attached to the handles. The locking mean may be attached to the base by any attachment means (such as pivot).
∘ clamping mean (2.4)

In one embodiment, a clamping means (or element) for a base of the flask is fitted within a hollow opening on the base of the flask holder and is arranged so that to maintain a base of the flask. The function of this clamping element is to maintain the flask blocked from rotation and vertical displacement. The clamping element may be attached to the base by any attachment means, such as a pivot.

In one embodiment, the flask is the Erlenmeyer flask, and the clamping element is a circular toothed spring with a horseshoe shape. The function of this element is to maintain the Erlenmeyer flask blocked from rotation and vertical displacement. The circular spring is arranged to contact (to latch) at least 60% of cylindrical Erlenmeyer flask base, when the clamping element is arranged around the Erlenmeyer flask base. The circular spring has additional toothed-shape embossment on the part of the edge and this embossment is contributing to maintain Erlenmeyer flask position. In one example, the toothed spring with a horseshoe shape (2.4) to maintain the Erlenmeyer can be seen as in Fig. 1B-D.

The circular spring has additional embossment on one or two ends of the spring referred herein a latching embossment (2.7). In one embodiment, the toothed clamping means have at least one, preferably at least two latching embossments.

The latching embossment is designed to latch to a safety latch (2.5). The latching embossment is contributing to the clamping of the flask. In one example, a latching embossment (2.7) that latches to a safety latch (2.5) can be seen as in Fig. 1C.
∘ a safety latch (2.5)

In one embodiment, a locking mean are exemplified by a latch further comprising at least one magnet inserted on the latch and by at least one countermagnet arranged on the base of the holder. The inserted at least one magnet may be glued on the latch and the at least one countermagnet may be glued on the base of the holder. The inserted at least one magnet may be a permanent magnet. In one embodiment, at least two permanent magnets may be used and inserted in the latch. In one embodiment, at least two countermagnets may be used and arranged on the flask holder base, so that the magnets are perpendicular to the base. In another exemplary embodiment two permanent magnets inserted in the latch and two countermagnets arranged on the base may be used, as seen in fig. 1C. In one embodiment, the magnets pairs are referred together as latching magnets (2.6). It is understood that at least one inserted magnet in the latch and at least one countermagnet arranged on the base are attached by magnetic attraction to each other when the safety latch is in the closed position as seen in fig. IB. Therefore, a set of the inserted magnets on the latch and the countermagnets on the base are providing additional latching means that secure toothed clamping means in the closed position as seen in fig. IB. The latch may be referred herein as a safety latch (2.5). The at least one magnet inserted on the latch and at least one countermagnet arranged on the base of the holder are referred herein as latching magnets (2.6) (fig. 1D-E).

In embodiment, the safety latch is made of hard anodized aluminium alloy.

In one embodiment the clamping mean is lifted away from the holder base so that the flask is placed on the holder's base. Next, the clamping mean is lowered and placed around the flask base. Finally, the safety latch is lifted so that at least one inserted magnet in the latch and at least one countermagnet arranged on the base can attach to each other and next the safety latch is secured with the latching embossment of the clamping means. In one example, the latching embossment (2.7) of the clamping means (2.4) may latch on the safety latch (2.5) when the clamping means, and the safety latch are in the closed position as seen in fig. IB.
∘ locking means for a bioreactor tube flask (10)

In one embodiment the locking means for a bioreactor tube (11) are arranged on the holder's base and perpendicular to the base's bottom. The locking mean may be attached to the base by any attachment means (such as screws).

In one embodiment the locking means for up to four bioreactor tubes (11) may comprise four guillotines (10.1), eight toothed jaws (10.2), four rail-and-pivots (10.3) (pivot not visible), a plunger (10.4), a plunger holder (10.5), an eccentric jig (10.6), a controlling latch (10.7).

In one embodiment the locking means for a bioreactor tube (11) comprise four guillotines (10.1) holding a toothed jaw (10.2). The four guillotines are driven by four rail-and-pivots (10.3) located in a plunger (10.4). The up and down movement of the plunger (10.4) is driven by an eccentric jig (10.6). This assembly (10) is connected to the controlling latch (10.7).

It is understood that up to four bioreactor tubes may be fitted into one flask holder.

It is understood that although exemplary alternative locking means for an Erlenmeyer flask and for bioreactor tubes are used, this do not influence other flask holder's elements technical specification and function thereof. Similarly, exemplary alternative locking means used in flask holder do not influence methods of flask holder's handling.

A flask holder with the locking means for a bioreactor tube is referred herein as a flask basket for bioreactor tubes (12).
- a magnetic pad (2.8)

In one embodiment, at least one, at least two, at least three, at least four countermagnets are arranged on the outer side of the flask holder base. Preferably, four countermagnets are arranged on the outer side of the flask holder base. In one embodiment, countermagnets are glued on the outer side of the flask holder base.

In one embodiment, the countermagnets are shaped as magnetic pads (2.8), as can be seen in Fig. IE.

In embodiment, the magnetic pads are made of magnetisable stainless steel (ferritic or martensitic). In general, use of stainless steel prevents from corrosion inside the incubator shaker compartment.

The flask holder horizontal position on the shaker tray is ensured by at least two conical positioning guiding elements present on the shaker tray and at least two magnets present on the shaker tray that can be attached by magnetic attraction to at least two magnetic pads arranged on the outer side of the flask holder base.

The flask holder horizontal position on the flask holder plate of the transfer shuttle is ensured by up to four conical positioning guiding elements present on the flask holder plate.

The function of these magnetic pads is to further maintain the flask holder vertical position.
- identification means (2.9)

In one embodiment, the flask holder is arranged with identification means, such as RFID (radio frequency identification) or two-dimensional barcode (e.g., QR-code) to enable culture information tracking (cell line I.D, passage number, passaging date, etc).

In one embodiment, the flask holder has RFID tag or QR-code (2.9). In one embodiment, a RFID tag or QR-code (2.9) may be arranged on the outer side of the handle, opposite to the gripping side, as can be seen in Fig. 1E with a QR-code (alternatively RFID tag could be located underneath the flask holder base).

The RFID is obtained by a reader/writer communicating with a PLC (Programmable Logic Controller) of an incubator shaker. The PLC then use this RFID information to generate a database to track the baskets during the use, the database may be used as a safety net in case of failure of the scheduling software, such as Hamilton scheduling software.

In one embodiment, a flask holder dimensions are adjusted to accommodate any flask.

In one embodiment, a flask to be placed within a holder is an Erlenmeyer flask, wherein a capacity of a flask may be selected from about 25 ml to 4000 ml, such as 25 ml, 50 ml, 125 ml, 250 ml, 500 ml, 1000 ml, 2000 ml or 4000 ml. In one embodiment, a flask is an Erlenmeyer flask, wherein a base diameter of a flask may be selected from about 4 cm to 20 cm, such as 4.1 cm, 5.1 cm, 6.7 cm, 8.2 cm, 10.1 cm, 12.9 cm, 16 cm or 20.6 cm. In one embodiment, a flask is an Erlenmeyer flask, wherein a high of a flask may be selected from about 6 cm to 36 cm, such as 6.1 cm, 7.8 cm, 11.4 cm, 13.2 cm, 17.6 cm, 21.6 cm, 26.8 cm or 36.0 cm.

In one embodiment, a flask is a 125 ml Erlenmeyer flask.

In one embodiment, a flask is 125mL Erlenmeyer flask (Thomson Optimum Growth^{™} 125mL Flask; reference 931110)).

In one embodiment, a flask to be placed within a holder is a bioreactor tube, wherein a capacity of a bioreactor tube may be selected from about 15 ml to 600 ml, such as 15 ml, 50 ml, 450 ml, or 600 ml. In one embodiment, a bioreactor tube can have conical or round base. In one embodiment, a flask is a bioreactor tube, wherein dimensions may be selected from 17.1×120mm, 30x115mm, 96×145mm and 98×183mm.

In one embodiment, a flask is a bioreactor tube (TPP TubeSpin^{®} Bioreactor 15), which can be conical on the bottom and allow smaller working volume of 1-15 ml.

In one embodiment, a flask has a closure cap (1.1; 11.1) having a top and adjoining a side wall of the flask along its circumferential periphery of a flask's neck. The closure cap is connected detachably to the flask. Good sealing of the interior space of the flask against the surrounding atmosphere and also secure fastening of the closure cap to the flask is, in an advantageous embodiment of the invention, achieved by the closure cap being configured as a screw closure. The closure cap may be a screw cap (1.1; 11.1) that may be a filtering cap. Such a cap has the advantage of providing for increased aeration while maintaining sterile conditions for cell cultures.

### An incubator shaker tray

In one embodiment, is provided an incubator tray (3) for holding at least one flask holder (2, 12) as described herein. The incubator tray (3) is suitable for use inside a shaker incubator and may be referred herein as an incubator shaker tray.

In one embodiment, the incubator tray may accommodate up to forty flask holders, such as forty flask holders for 125ml Erlenmeyer flasks arranged in 8x5 array as seen on Fig. 2C. The function of the incubator tray is to maintain flask holders in a stable position while a shaker incubator is operating.

In one embodiment, a shaker tray is preferably made of anodized hard anodized aluminium alloy. This prevents the tray from corrosion inside the incubator shaker compartment.

In one embodiment, is provided an incubator tray (3) that comprises a lower plate (3.1), an upper plate (3.2) and a tray locker (3.3).

Possible elements of the incubator tray may be described as follows:
- a lower plate (3.1)

In one embodiment, a lower plate (3.1) is arranged below an upper plate (3.2) and is attached to the upper plate (3.2) by any attachment means, preferably by screws.

In one embodiment, the lower plate is preferably made of hard anodized aluminium alloy.

In one embodiment, the lower plate is about 3 mm to about 5 mm thick, preferably about 4 mm thick.

In one embodiment, the lower plate (3.1) has up to eighty electro-magnets arranged on the top of a lower plate. The electro-magnets may be screwed on the top of a lower plate. In one embodiment, permanent electro-magnets (3.4) may be used.

The permanent electro-magnets (3.4) are guided into the upper plate (3.2) through bores. The permanent electro-magnets' magnetic field is suppressed when a coil is activated. The permanent electro-magnets are divided into eight sections within the tray, each one controlled by a solid-state relay. Relays are controlled through controller means such as an I2C bus by a microcontroller (such as Arduino controller) communicating to the Programmable Logic Controller (PLC) (such as Festo) through an ME3/ME4 control card (such as Coppley control). In one embodiment, the lower plate (3.1) has up to forty RFID reader/writer arranged on the top of the lower plate. RFID reader/writer are communicating by I2C protocol to a microcontroller (such as Arduino). RFID reader/writer may include a build-in antenna.

In one embodiment, the lower plate is shown as on Fig. 1A. In one embodiment, the lower plate assembled with the upper plate is shown on Fig. IB.
- an upper plate (3.2)

In one embodiment, an upper plate (3.2) is arranged above a lower plate (3.1) and is attached to the lower plate (3.1) by any attachment means, preferably by screws and maintained together at a distance through spacers. In one embodiment, a distance between a lower plate (3.1) and an upper plate (3.2) arranged above is about 10 mm to about 11 mm, preferably about 10.5 mm.

In one embodiment, the upper plate is preferably made of hard anodized aluminium alloy.

In one embodiment, the upper plate is about 3 mm to about 5 mm thick, preferably about 3 mm thick.

In one embodiment, the upper plate has at least one guiding element arranged on the top of the upper plate. In one embodiment, the upper plate has up to eighty guiding elements arranged on the top of the upper plate. The guiding elements may be attached to the top of the upper plate by any attachment means, preferably by screws. The guiding element are referred herein as centering guiding element or positioning guiding element. The guiding element may be arranged so that two guiding elements are arranged for each flask basket location. The centering guiding element may have conical shape. The centering guiding elements may be exemplified by pins, such as centering pins (3.5).

In one embodiment, the flask holder is placed between two guiding elements present on the incubator tray and this provides a horizontal position for the flask holder.

In one embodiment, an upper plate (3.2) has up to forty holes for RFID reader/writer antennas, referred herein as RFID antennas holes (3.6).

It is understood that countermagnets on the flask holder (2.8) and electromagnets (3.4) on the tray are coincident through the guiding function of the guiding elements (such as centering pins (3.5)) arranged on the upper plate (3.2).
- a tray locker (3.3)

In one embodiment, a tray locker (3.3) is arranged on an orbital tray holder.

In one embodiment, a tray locker (3.3) provides precise positioning of the tray by two ways. First, by a two half-moon milling on the tray corresponding to two cylindrical jigs in the back side of the tray. Second, in the front side of the tray, by an eccentric handle and blocking the tray in a fixed and precise position.

In one embodiment, a tray locker (3.3) is compatible with automation as described herein.

### 3-axis motorized stage

In one embodiment, is provided a 3-axis motorized stage (4) that is designed to be mounted on the inner side of a top wall (a ceiling) of an incubator shaker chamber. The 3-axis motorized stage is attached to the incubator shaker chamber by any attachment means, preferably by stainless steel threaded dowels and non-heat conductive *PEEK* nuts.

It will be appreciated that a 3-axis motorized stage may be a compact system (with double extension) that allows to increase the operational height of the gripping system. Such a compact (low height) 3-axis motorized stage allow the handling and transportation of high flask holder such as a flask holder for an Erlenmeyer flask or a bioreactor tube. In one embodiment, a payload capacity of a 3-axis motorized stage is 0.8 kg.

In one embodiment, is provided a 3-axis motorized stage (4) that comprises X-axis (4.1), Y-axis (4.2), Z-axis (4.3), and at least one motor for driving each axis. In one embodiment a motor for driving an X-axis or Y-axis or Z-axis (4.3) is a linear motor. In one embodiment, is provided at least one encoder that provides feedback for close-loop control of at least one motor for driving each axis.

In one embodiment, is provided a 3-axis motorized stage (4) that comprises X-axis (4.1), Y-axis (4.2), Z-axis (4.3), a linear motor (4.4) for driving the X-axis (4.1), a linear motor (4.5) for driving the Y-axis (4.2), a linear motor (4.6) for driving the Z-axis (4.3) and means for gripping a flask holder (4.7), as seen on Fig. 3.

Possible elements of the 3-axis motorized stage may be described as follows:
- X-axis (4.1)

In one embodiment, X-axis has about 685 mm travel range. Preferably, the 3-axis motorized stage has one X-axis.

In one embodiment, the X-axis (4.1) is connected to one linear motor (4.4).
- Y-axis (4.2)

In one embodiment, Y-axis has about 375 mm travel range. Preferably, the 3-axis motorized stage has one Y-axis.
- Z-axis (4.3)

In one embodiment, Z-axis has about 175 mm travel range. Preferably, the 3-axis motorized stage has one Z-axis.
- a linear motor (4.4) for driving the X-axis (4.1)

In one embodiment, the X-axis (4.1) is connected to one linear motor (4.4) so that the linear motor drives the X-axis. In one embodiment, the linear motor driving the X-axis is selected from linear motors known in the art, such as TM6 linear motor (Technotion).

In one embodiment, is provided an LA11 absolute encoder (RLS) that provides feedback for close-loop control of the linear motor (4.4) through the ME3 card.
- a linear motor (4.5) for driving the Y-axis (4.2)

In one embodiment, the Y-axis (4.1) is connected to one linear motor (4.5) so that the linear motor drives the Y-axis. In one embodiment, the linear motor driving the Y-axis is selected from linear motors known in the art, such as TM6 linear motor (Technotion).

In one embodiment, is provided an LA11 absolute encoder (RLS) that provides feedback for close-loop control of the linear motor (4.5) through the ME3 card.
- a linear motor (4.6) for driving the Z-axis (4.3)

In one embodiment, the Z-axis (4.3) is connected to one linear motor (4.6) so that the linear motor drives the Z-axis. In one embodiment, the linear motor driving the Z-axis is selected from linear motors known in the art, such as TM3 linear motor (Technotion). Z-axis provides extension movement.

In one embodiment, is provided an LA11 absolute encoder (RLS) that provides feedback for close-loop control of the linear motor (4.6) through the ME3 card.

In one embodiment, the linear motors may be placed within a case (as seen in Fig. 3).
- means for gripping a flask holder

In one embodiment, are provided means for gripping a flask holder. The means for gripping a flask holder are attached to Z-axis (4.3) by any attachment means, such as screws. The function of these gripping means is to allow gripping of a flask holder for transportation. The function of the gripping means is to allow for detachable connection to a flask holder. The gripping means must be thus compatible with a flask holder handles.

In one embodiment, an example of means for gripping a flask holder is one parallel gripper (4.7) with an adjustable prehension force, equipped with jaws (4.8) and fingers (4.9) for detachable connection to a flask holder. In one embodiment, a parallel gripper is selected from parallel gripper known in the art, such as an electric EHPS parallel gripper (Festo).

In embodiment, the parallel gripper is made of hard anodized aluminium alloy.

In embodiment, the parallel gripper' jaws are made of stainless steel.

In embodiment, the parallel gripper' fingers are made of hard anodized aluminium alloy.

In embodiment, the parallel gripper is controlled by the PLC, communicating via IO-Link interface.

### A sliding door

In one embodiment, is provided a sliding door (5) that is designed to be mounted within a side wall of an incubator shaker chamber. The sliding door (5) is designed to be mounted within a rounded-rectangular hole drilled on side wall of an incubator shaker chamber. Preferably, the sliding door (5) is designed to be mounted on a left side wall of an incubator shaker chamber. In one embodiment, the sliding door is automated. In one embodiment, the sliding door is attached to the incubator shaker chamber by any attachment means, preferably by screws. In one embodiment, the sliding door comprises a door frame (5.1) and a door panel (5.2), and when the door panel is open it makes a door tunnel (5.3).

In another further embodiment, the door panel (5.2) consists of three parts that are front panel (5.2.1) (fig. 4B), main body (5.2.2) (fig. 4C) and pressing panel (5.2.3) (fig. 4D).

In one embodiment, is provided the sliding door (5) that comprises a door frame (5.1) and a door panel (5.2) and at least one motor for driving the door movement. In one embodiment, is provided the sliding door (5) that comprises a door frame (5.1) and a door panel (5.2), sliding movement driving stepper motor (5.4) and press movement driving stepper motor (5.5). The sliding door may further comprise at least one sensor for door movement.

The sliding door may further comprise heating means, for example comprising a temperature probe (5.8), a heating pad (5.9), a heating ribbon (5.11) and a controller. The sliding door may further comprise a precision sensor for sliding movement (5.6) and a precision sensor for press movement (5.7), a temperature probe (5.8), a heating pad (5.9), an o-ring (5.10) and a heating ribbon (5.11), as seen on Fig. 4.

Possible elements of the sliding door may be described as follows:
- a door frame (5.1)
- a door panel (5.2)

The door panel of the door slides to open and close. When the door panel is open it creates a door tunnel (5.3). The door tunnel is the space through which items are moved when the door is open.

The door panel (5.2) consists of three parts that are a front panel (5.2.1) (fig. 4B), a main body (5.2.2) (fig. 4C) and a pressing panel (5.2.3) (fig. 4D). In another embodiment, a front panel (5.2.1) is arranged with sliding movement driving stepper motor (5.4) and press movement driving stepper motor (5.5). In one embodiment, a main body (5.2.2) is arranged between a front panel (5.2.1) and a pressing panel (5.2.3). In one embodiment, a pressing panel (5.2.3) is arranged with a temperature probe (5.8) and a heating pad (5.9), preferably said temperature probe and said heating pad are embedded in the pressing panel. In one embodiment, when the door is closed, a pressing panel (5.2.3) is in contact with an o-ring (5.10). The sliding door exert pressure on the o-ring.
- two stepper motors: sliding movement driving stepper motor (5.4) and press movement driving stepper motor (5.5)

In one embodiment, the sliding door comprises at least one motor for driving the door movement, such as sliding movement driving stepper motor (5.4) and press movement driving stepper motor (5.5). In one embodiment, the stepper motors are selected from stepper motors known in the art, such as NEMA-23. In one embodiment, the stepper motors are controlled by controller means such as the ME4 control card.

In one embodiment, one stepper motor is driving the sliding movement (5.4) through a simple rack and pinion. In one embodiment, one stepper motor driving the sliding movement is located on the right side of the door, such as on the front panel (5.2.1).

In one embodiment, one stepper motor is driving the press movement (5.5) through a motor gearbox, two toothed wheel and four satellite wheels. The four satellite wheels drive four leadscrews which control the press movement. Two sliding ball-bearing guide the global mechanism.

In one embodiment, one stepper motor driving the sliding movement is located on the left side of the door, such as on the front panel (5.2.1).
- two sensors: a precision sensor for sliding movement (5.6) and a precision sensor for press movement (5.7)

In one embodiment, the sliding door comprises at least one sensor for door movement, such as sensor for sliding movement (5.6) and a sensor for press movement (5.7). In one embodiment, the sensors are precision sensors. In one embodiment, the precision sensors are selected from sensors known in the art, such as precision sensor switch (Baumer). In one embodiment, the sensors are controlled controller means, such as by the ME4 control card, the last being controlled by the PLC.

In one embodiment, one precision sensor is dedicated to the detection of the sliding movement when the door is opened (no detection when the door is closed). It is located on the door frame (5.1). It is referred herein as a precision sensor for the homing of the sliding movement (5.6). In one embodiment, one precision sensor is dedicated to the detection of the press movement. It is located on the sliding door panel (5.2). It is referred herein as a precision sensor for the homing of the press movement (5.7), when the door is unpressed (such as opened door).
- a temperature probe (5.8)

In one embodiment, a temperature probe is selected from temperature probes known in the art, such as Pt1000 probe (5.8). The used temperature probe is suitable for precision temperature sensing. In one embodiment, at least one temperature probe is used. It is located on the door, such as embedded in the pressing panel (5.2.3).

In one embodiment, the temperature probe is connected to a microcontroller (such as Arduino controller) controlling a heating pad (5.9) and/or a heating ribbon (5.11). The microcontroller may communicate the door temperature value to the PLC for temperature monitoring.
- a heating pad (5.9)

In one embodiment, a heating pad is selected from heating pads known in the art, such as polyimide encapsuled heating pad (5.9). In one embodiment, at least one heating pad is used. In one embodiment, one polyimide encapsuled heating pad is embedded in the door, such as embedded in the pressing panel (5.2.3). Use of a heating pad enable the door temperature control and prevent condensation on door surface.
- an o-ring (5.10)

In one embodiment, an o-ring is selected from an o-ring known in the art, such as silicone o-ring (5.10). An o-ring may be referred to as a countour ring. In one embodiment, at least one o-ring is used. In one embodiment, one silicone o-ring is fitted between the door frame (5.1) and the pressing panel (5.2.3) of the door panel (5.2). Use of an o-ring ensures air-proofing.
- a heating ribbon (5.11)

In one embodiment, a door frame (5.1) is arranged with at least one heating ribbon (5.11) located around the opening creating the door tunnel (5.3).

In one embodiment, a heating ribbon is selected from heating ribbon known in the art, such as polyimide encapsuled heating ribbon. In one embodiment, at least one heating ribbon is used. Use of a heating ribbon enable the tunnel temperature control and prevent condensation on tunnel surface.

### A transfer shuttle

In one embodiment, is provided a transfer shuttle (6) that is designed to be mounted on the outer side of a side wall of an incubator shaker chamber. Preferably, the transfer shuttle (6) is designed to be mounted on a left side wall of an incubator shaker chamber. In one embodiment, the transfer shuttle is automated.

In one embodiment, the transfer shuttle is attached to the incubator shaker chamber by any attachment means, preferably by screws.

The transfer shuttle (6) is designed to be mounted on the outer side of a side wall of an incubator shaker chamber below the sliding door (5), as seen on Fig. 6. This allows movement of a flask holder plate (6.5) through the sliding door tunnel (5.3) created when the sliding door panel (5.2) is open.

In one embodiment, the automated transfer shuttle (6) may comprise at least a sliding frame (such as 6.1), rails (such as 6.2), at least two belts (such as 6.11) arranged on the sliding frame, a cell culture flask holder plate (such as 6.5) and a motor for driving sliding movement of the plate on the rails (such as 6.3).

In one embodiment, the automated transfer shuttle (6) may comprise at least a sliding frame (6.1) and rails (6.2), sliding movement driving stepper motor (6.3), a ball screw (6.13), a flask holder plate (6.5), belts (6.11), a flask holder plate (6.5) with two guiding elements (6.6). The automated transfer shuttle (6) may further comprise a pinion (6.4), an amplified precision sensor for detecting the transfer shuttle home position (6.7), a sensor for detecting of the loading position of the flask holder (6.8) and a sensor for detecting of the unloading position of the flask holder (6.9) and a presence laser detector (6.10), as seen on Fig. 5.

Possible elements of the transfer shuttle (6) may be described as follows:
- a sliding frame (6.1) and rails (6.2) and a sliding movement driving motor (6.3)

In one embodiment, at least one frame is used. In one embodiment, the frame is made of stainless-steel.

In one embodiment, the sliding frame (6.1) holds at least two rails. In another exemplary embodiment, the sliding frame (6.1) holds four rails, two upper and two lower (6.2). The upper plate may be sliding on the upper rails. The sliding frame slides on the shuttle base through the lower rails.

In one embodiment, a sliding movement driving motor is a stepper motor (6.3).

The sliding is controlled by a stepper motor (6.3) through a ball screw (6.13) (first movement) (Fig. 5). In one embodiment, the stepper motor is selected from stepper motors known in the art, such as NEMA-23. In one embodiment, one stainless-steel frame is sliding on rails, wherein sliding moved is controlled by a NEMA-23 stepper motor through a ball screw (6.13) (first movement). In one embodiment, one stepper motor that is driving the sliding movement is located below the rails.

In one embodiment, the stepper motor is controlled by a controller means, such as by the ME4 control card, the last controlled by the PLC.
- a pinion (6.4)

In one embodiment, the pinion (6.4) is attached on the sliding frame (6.1) by a pinion holder screwed on it. In one embodiment, the pinion (6.4) is driving the double extension of the upper plate (6.12) through racks (second movement).
- flask holder plate (6.5)

In one embodiment, at least one flask holder plate is used. The flask holder plate is sliding on rails (6.2). The sliding movement of the flask holder plate (6.5) on rails (6.2) is driven by at least two belts (6.11) arranged on a sliding frame (6.1) (third movement). In another embodiment, the sliding movement of the flask holder plate (6.5) on rails (6.2) is driven by at least four belts (6.11) arranged on a sliding frame (6.1) (third movement). In one embodiment, belts are glued and screw-pinched by one side on the sliding frame and by the other side on the flask holder. A tensioner is installed at one side. Pulley installed on the double extension plate (6.12) inverts the movement direction. The belts are preferably made of polyimide film. In general, use of polyimide film provides more mechanical resistance (no stretching) and since it is thinner than other common materials used for belts it allows for more compact belts while maintaining their resistance.

In one embodiment, the flask holder plate (6.5) comprises at least two guiding elements attached to at the top of the flask holder plate by any attachment means, preferably by screws. The guiding element is referred herein as centering guiding element or positioning guiding element. Preferably, four centering guiding elements are arranged for a flask holder. The guiding element may have conical shape. The centering guiding elements may be exemplified by pins, such as centering pins (6.6). In one example, the flask holder is placed between at least two or at least four guiding elements (such as pins) present on the flask holder plate and this provides a horizontal position for a flask holder.
- position sensors/detectors comprising an amplified precision sensor for detecting the transfer shuttle home position (6.7), a precision sensor for detecting of the loading position of a flask holder (6.8), a precision sensor for detecting of the unloading position of a flask holder (6.9)

In one embodiment, the transfer shuttle comprises at least one sensor for detecting the transfer shuttle home position, such as a home position and/or a loading position of a flask holder and/or unloading position of a flask holder and are thus preferably referred herein as position sensors/detectors. In one embodiment, the sensors are selected from an amplified precision sensors or precision sensors.
∘ an amplified precision sensor for detecting the transfer shuttle home position (6.7)

In one embodiment, a sensor for detecting the transfer shuttle home position (6.7) may be an amplified precision sensor. In one embodiment, the amplified precision sensor is selected from sensors known in the art, such as precision sensor switch (Baumer). In one embodiment, the sensor is controlled by controller means such as the ME4 control card, this later being controlled by the PLC.

In one embodiment, preferably at least one amplified precision sensor is dedicated for detection of the home position of the transfer shuttle. It is located at the shuttle frame and detecting the position of the sliding frame. It is referred herein as a precision sensor for detecting the transfer shuttle home position (6.7). The home position is shown in Figure 5 or 6 and is representing the idle status, defined as a non-extended state with the flask holder plate located outside of the incubator compartment.
∘ two precision sensors for detecting of the loading/unloading position of the flask holder (6.8) and (6.9)

In one embodiment, a sensor for detecting of the loading/unloading position of the flask holder (6.8) and (6.9) may be a precision sensor. In one embodiment, the precision sensor is selected from sensors known in the art, such as precision sensor switch (Metrol). In one embodiment, the sensor is controlled by controller means such as the ME4 control card, this later being controlled by the PLC.

In one embodiment, preferably at least one precision sensor is dedicated for detection of the loading position of the flask holder, i.e., when the flask holder is placed on the flask holder plate inside the incubator shaker compartment. In one embodiment, preferably at least another precision sensor is dedicated for detection of the unloading position of the flask holder, i.e., when the flask holder is removed from the flask holder plate outside the incubator shaker compartment. The two sensors are referred herein as a sensor for detecting of the loading position of the flask holder (6.8) and a sensor for detecting of the unloading position of the flask holder (6.9). The two sensors for detecting of the loading/unloading position of the flask holder are located at opposite sides of the the double extension plate (6.12). The loading position represents the status when the transfer shuttle is extended with the flask holder plate located within the incubator shaker compartment. The unloading position corresponds to the home position as shown in Figure 5 or 6 and defined as a non-extended state with the flask holder plate located outside of the incubator shaker compartment.
∘ a laser detector (6.10)

The laser detector allows to detect presence or absence of a flask holder (basket) and is thus preferably referred herein as a presence laser detector. In one embodiment, a presence laser detector is selected from presence laser detector known in the art.

The preferably used presence laser detector is suitable to measure distance, such as a distance between basket on the shuttle inside the incubator and basket outside the incubator. If a flask holder is present, the presence laser detector allows to identify whether the flask holder is grippible inside/outside the incubator. In one embodiment, at least one presence laser detector is used. The presence laser detector may be located on one external extremity of the transfer shuttle. The presence laser detector may be directly controlled by a controller such as by the PLC, communicating via IO-Link interface.

### An automated incubator shaker compartment

In one embodiment, is provided an assemble of elements comprising
- an incubator shaker tray (3),
- a 3-axis motorized stage (4),
- a sliding door (5), and
- a transfer shuttle (6),
designed to be arranged in an incubator shaker chamber. This assemble of elements is essential to maintain and then to transport at least one cell culture flask arranged in at least one cell culture flask holder and thus is referred herein as a set or alternatively as a device. In one embodiment, an incubator shaker tray function to maintain at least one cell culture flask holder. In one embodiment, a 3-axis motorized stage function is to first maintain and then simultaneously maintain and transfer one cell culture flask holder. In one embodiment, a sliding door function is to allow transfer of one cell culture flask holder inside/outside an incubator shaker chamber. In one embodiment, a transfer shuttle function is to first maintain and then simultaneously maintain and transfer one cell culture flask holder.

In one embodiment, is provided a system comprising
- an incubator shaker tray (3),
- a 3-axis motorized stage (4),
- a sliding door (5), and
- a transfer shuttle (6),
for an incubator shaker chamber.

In one embodiment, the incubator shaker tray (3), the 3-axis motorized stage (4), the sliding door (5) or the transfer shuttle (6) as described herein may be used individually and be assembled with any other compatible devices.

In one embodiment is provided a set (7) for maintaining and transporting at least one cell culture flask, the set is suitable for an incubator shaker compartment, and is comprising
- an incubator shaker tray (3),
- a 3-axis motorized stage (4),
- a sliding door (5), and
- a transfer shuttle (6),
wherein the at least one cell culture flask is arranged in at least one cell culture flask holder (2, 12) compatible with the set (7).

In one embodiment the set is an automated set. In one embodiment the set (7) for maintaining and transporting cell culture flasks suitable for an incubator shaker compartment may be referred to as an automated incubator shaker compartment.

In one embodiment, the set (7) is designed to be mounted to an incubator shaker chamber so that
- the incubator shaker tray (3) is designed to be mounted inside the incubator shaker chamber,
- the 3-axis motorized stage (4) is designed to be mounted on the inner side of a top wall of the incubator shaker chamber,
- the sliding door (5) is designed to be mounted within a rounded-rectangular hole on the side wall of an incubator shaker chamber,
- the transfer shuttle (6) is designed to be mounted on the outer side of a side wall of an incubator shaker chamber.

The above exemplary design of the set (7) to the incubator shaker chamber is shown on Fig. 7. In one embodiment the parts of the set (7) of the invention comprising
- an incubator shaker tray (3),
- a 3-axis motorized stage (4),
- a sliding door (5), and
- a transfer shuttle (6),
have at least one feature or any combination of features as described herein.

In one embodiment the at least one cell culture flask holder (2, 12) compatible with the set (7) have at least one feature or any combination of features as described herein.

In one embodiment, the set of the invention is suitable to be fitted to any incubator shaker, and in particular an incubator shaker having the dimension selected from about 1288 mm length (L), about between 859 to 917 mm depth (D), and about 771 mm hight (H).

In one embodiment, the set of the invention is fitted to the incubator shaker having the chamber with the dimension selected from 1288 × 859 × 771 mm (L × D × H) or 1288 × 917 × 771 mm (L × D × H) or 1288 × 887 × 771 mm (L × D × H) or 1288 × 859 × 771 mm (L × D × H), preferably 1288 × 859 × 771 mm (L × D × H).

### An incubator shaker with an automated incubator shaker compartment

In one embodiment is provided an incubator shaker comprising a set, a device, a system or an assemble of elements as described herein.

In one embodiment is provided an incubator shaker (8) comprising a set (7) for maintaining and transporting at least one cell culture flask, the set comprising
- an incubator shaker tray (3),
- a 3-axis motorized stage (4),
- a sliding door (5), and
- a transfer shuttle (6),
wherein at least one cell culture flask is arranged in at least one cell culture flask holder (2, 12) compatible with the set (7).

In one embodiment the set in the incubator shaker is an automated set. In one embodiment the set in the incubator shaker may be referred to as an automated incubator shaker compartment.

In one embodiment, is provided an incubator shaker (8) comprising a set (7), wherein the set is designed to be mounted to an incubator shaker chamber so that
- the incubator shaker tray (3) is designed to be mounted inside the incubator shaker chamber,
- the 3-axis motorized stage (4) is designed to be mounted on the inner side of a top wall of the incubator shaker chamber,
- the sliding door (5) is designed to be mounted within a rounded-rectangular hole on the side wall of an incubator shaker chamber,
- the transfer shuttle (6) is designed to be mounted on the outer side of a side wall of an incubator shaker chamber.

In one embodiment, the sliding door (5) is designed to be mounted within a rounded-rectangular hole drilled on the side wall of an incubator shaker chamber.

In one embodiment is provided an incubator shaker (8) comprising a set (7), wherein the parts of the set (7) of the invention comprising
- an incubator shaker tray (3),
- a 3-axis motorized stage (4),
- a sliding door (5), and
- a transfer shuttle (6),
have at least one feature or any combination of features as described herein.

In one embodiment the at least one cell culture flask holder (2, 12) compatible with an incubator shaker (8) comprising a set (7) have at least one feature or any combination of features as described herein.

In one embodiment, the incubator shaker comprising a set of the invention may be any incubator shaker, and in particular the incubator shaker having the dimension selected from about 1288 mm length (L), about between 859 to 917 mm depth (D), and about 771 mm hight (H).

In one embodiment, the incubator shaker comprising a set of the invention has the chamber with the dimension selected from 1288 × 859 × 771 mm (L × D × H) or 1288 × 917 × 771 mm (L × D × H) or 1288 × 887 × 771 mm (L × D × H) or 1288 × 859 × 771 mm (L × D × H), preferably 1288 × 859 × 771 mm (L × D × H).

In one embodiment is provided the incubator shaker comprising a set of the invention, wherein the incubator shaker is a part of an automated cell culturing platform, such as an automated passaging platform.

The exemplary arrangement of the incubator shaker (8) comprising a set of the invention (7) with an automated cell passaging platform is shown on Fig. 8.

In one embodiment, a chamber of a liquid handling system (9) has a hole that is following the contours of the automated sliding door (5) of the incubator shaker (8), and the the incubator shaker (8) is designed so that the flask holder (2, 12) may be transferred on the transfer shuttle (6) to the liquid handling system (9).

In one embodiment provided is a use of the incubator shaker comprising a set of the invention for cell culturing, in particular for cell passaging.

In one embodiment provided is a method of cell culturing comprising a use of the incubator shaker comprising a set of the invention.

### Automated procedures of an automated incubator shaker

It is understood that flask holders (2, 12) may be placed in and removed from an incubator shaker (8) through a main front door of an incubator shaker (8).

The non-automated steps for the flask holders (2, 12) handling/loading may include the following steps:
a) empty holder position (unlocked safety latch),
b) flask drop off within the flask holder,
c) safety latch locking,
d) incubator shaker opening through the main front door,
e) holder loading on the incubator tray; holder positioning on the tray through two pins and basket locking on the tray through magnetic attraction,
f) RFID detection and verification,
g) incubator shaker closing through the main front door.

It is understood that flask holders (2, 12) may be placed in and removed from an incubator shaker (8) through a sliding door (5) of an incubator shaker (8).

The automated steps for the flask holders (2, 12) transporting may include:
a) 3-axis stage at home position,
b) X/Y axis stage movement above holder pick-up position,
c) Z axis stage movement to holder pick-up position,
d) holder gripping through opening of the gripper fingers,
e) unactivation of the holder electro-magnets,
f) holder pick-up through Z axis stage movement,
g) X/Y axis stage movement above shuttle position,
h) sliding door opening and shuttle extension towards shaker incubator compartment (loading position),
i) holder loading on the shuttle through Z axis stage movement,
j) shuttle back at home position and sliding door closing back at home position,
k) X/Y/Z axis stage back at home position.

In the case of parallel cultivation of a plurality of cultures in one shaker incubator, such an automated procedure for transporting one flask holder via the set of the invention allows to limit negative changes on the environment of the cultures in the other labware.

In one embodiment, is provided a method for maintaining at least one cell culture flask arranged in at least one cell culture flask holder with a set as described herein.

In one embodiment, is provided a method for maintaining and transporting at least one cell culture flask arranged in at least one cell culture flask holder with a set as described herein.

### An automated cell passaging platform comprising an incubator shaker with an automated incubator shaker compartment

In one embodiment is provided an automated cell passaging platform comprising a liquid handling system, at least one robotized arm, at least one automated hotel, an automated refrigerated incubator, an automated microplate centrifuge with automated loader, a cell counting device and at least one shaker incubator, and optionally an automated orbital shaker incubator, characterised in that
the at least one shaker incubator is designed to be mounted with the set for maintaining and transporting at least one cell culture flask as described herein.

In one embodiment, is provided an automated cell passaging platform as described herein, characterised in that it comprises at least two shaker incubators, and
the at least two shaker incubators are designed to be mounted with the set for maintaining and transporting cell culture flasks as described herein.

It is understood that the capacity of an automated cell passaging platform is up to eighty cell culture flasks such as Erlenmeyer flasks, i.e., up to forty in each shaker incubator. It is understood that the capacity of an automated cell passaging platform is up to 320 bioreactor tubes, i.e., up to 160 in each shaker incubator.

In one embodiment, a chamber of the liquid handling system (9) has a hole that is following the contours of the automated sliding door (5) of the incubator shaker (8), and the at least one incubator shaker (8) is designed so that the flask holder (2, 12) may be transferred on the transfer shuttle (6) to the liquid handling system (9).

In one embodiment provided is a use of an automated cell passaging platform comprising at least one incubator shaker with an automated incubator shaker compartment as described herein for cell passaging.

In one embodiment provided is a method of cell culturing comprising a use of an automated cell passaging platform comprising at least one incubator shaker with an automated incubator shaker compartment as described herein.

References cited herein are hereby incorporated by reference in their entirety. The present invention is not to be limited in scope by the specific embodiments and drawings described herein, which are intended as single illustrations of individual aspects of the invention, and functionally equivalent methods and components are within the scope of the invention. The examples illustrating the invention are not intended to limit the scope of the invention in any way.

## Claims

1. A set for maintaining and transporting at least one cell culture flask arranged in at least one cell culture flask holder, wherein the set is designed to be arranged in an incubator shaker chamber, and the set comprising
- an incubator shaker tray designed to be mounted on the inside of the incubator shaker chamber, that comprises at least one guiding element arranged for maintaining each cell culture flask holder,
- a 3-axis motorized stage designed to be mounted on the inner side of a top wall of the incubator shaker chamber, that comprises X-axis, Y-axis and Z-axis and three linear motors for driving the three axis and means for gripping the cell culture flask holder,
- a sliding door designed to be mounted within a side wall of the incubator shaker chamber, that comprises a door panel and at least one motor for driving the door movement,
- a transfer shuttle designed to be mounted on the outer side of a side wall of the incubator shaker chamber, that comprises a sliding frame, rails, at least two belts arranged on the sliding frame, a cell culture flask holder plate and a motor for driving sliding movement of the plate on the rails.

2. The set for maintaining and transporting at least one cell culture flask according to claim 1, wherein the cell culture flask is a 125ml Erlenmeyer flask or a bioreactor tube.

3. A set according to claim 1 or 2, wherein the means for gripping the cell culture flask holder are attached to Z-axis of the 3-axis motorized stage and comprise one parallel gripper with an adjustable prehension force, equipped with jaws and fingers for detachable connection to the flask holder.

4. A set according to claim 1,2 or 3, wherein the motor for driving the door movement of the sliding door, comprises a stepper motor for driving a sliding movement and a stepper motor for driving a press movement of the door panel.

5. A set according to any of the claims 1 to 4, wherein the sliding door further comprises a precision sensor for detecting a sliding movement of the door panel and a precision sensor for detecting press movement of the door panel.

6. A set according to any of the claims 1 to 4, wherein the sliding door further comprises a temperature probe, a heating pad, a heating ribbon, and a controller, and wherein the temperature probe is controlling the heating pad and the heating ribbon through the controller.

7. A set according to any of the claims 1 to 6, wherein the sliding door further comprises a silicone o-ring fitted between the door frame and the door panel.

8. A set according to any of the claims 1 to 7, wherein the transfer shuttle further comprises an amplified precision sensor for detecting a transfer shuttle home position, a sensor for detecting of loading position of the cell culture flask holder, and a sensor for detecting of unloading position of the cell culture flask holder.

9. A set according to any of the claims 1 to 8, that further comprises a cell culture flask holder, and the cell culture flask holder comprises a base, two handles arranged perpendicular to the base and opposite of each other, a locking mean for maintaining the cell culture flask and a heat conductive pad arranged on the base of the holder.

10. A set according to claim 9, wherein the cell culture flask holder further comprises at least two magnets arranged on the outer side of the cell culture flask holder base that provide attachment means to the incubator shaker comprising permanent electromagnets.

11. A set according to claim 9, wherein the cell culture flask holder's locking mean comprises a clamping mean, and a safety latch arranged on the cell culture flask holder base.

12. A set according to claim 9, wherein the cell culture flask holder's locking mean comprises four guillotines each holding a toothed jaw and arranged on the cell culture flask holder base.

13. Use of the set according to any of the claims 1-12 for maintaining and transporting at least one cell culture flask arranged in at least one cell culture flask holder compatible with the set.

14. An incubator shaker comprising the set according to claim 1.

15. An automated cell passaging platform comprising a liquid handling system, at least one robotized arm, at least one automated hotel, an automated refrigerated incubator, an automated microplate centrifuge with automated loader, a cell counting device and at least one shaker incubator, and optionally an automated orbital shaker incubator,
**characterised in that**
the at least one shaker incubator is designed to be arranged with the set for maintaining and transporting at least one cell culture flasks according to claim 1.
